# EUROPEAN PATENT SPECIFICATION

(11) **EP 4 530 976 B1**
(45) Date of publication and mention of the grant of the patent: **22.04.2026**
(21) Application number: 23465538.9
(22) Date of filing: 26.09.2023
(51) Int. Cl.: G06T 7/00, G16H 30/20, G16H 30/40, G16H 50/20, G16H 50/30, A61B 6/50

(54) **CORONARY ARTERY LUMEN CONTOUR ADJUSTMENT BASED ON SEGMENTATION UNCERTAINTY**
KONTURANPASSUNG DES KORONARARTERIENLUMENS AUF BASIS VON SEGMENTIERUNGSUNSICHERHEITEN
AJUSTEMENT DU CONTOUR DE LA LUMIÈRE D'UNE ARTÈRE CORONAIRE SUR LA BASE D'UNE INCERTITUDE DE SEGMENTATION

(43) Date of publication of application: 02.04.2025
(73) Proprietor: Siemens Healthineers AG, 91301 Forchheim (DE)
(72) Inventor: NEUMANN, Dominik, 91052 Erlangen (DE); TURCEA, Alexandru, 105500 Busteni, Prahova (RO); ITU, Lucian Mihai, 500294 Brasov (RO); CIMEN, Serkan, Jersey City, NJ, 07310 (US)
(74) Representative: Kraus & Lederer PartGmbB

(56) References cited:
- EP-A2- 2 966 615
- US-A1- 2015 272 448
- US-A1- 2018 344 173

## Description

### TECHNICAL FIELD

Various examples of the disclosure generally relate to angiography. Various examples specifically relate to processing multiple cardiac images depicting a portion of coronary arteries within an anatomical region of interest to adjust one or more segments of a contour of a given lumen segmentation of the portion of the coronary arteries.

### BACKGROUND

Extraction or segmentation of vessel lumen from cardiac angiography images, e.g., X-ray angiography images or computed tomography angiography (CTA) images, is an integral part of angiography processing pipelines for extracting determining or calculating measurements such as minimal lumen diameter, stenosis severity, etc., or more advanced ones such as virtual fractional flow reserve (FFR), where a contour of a lumen segmentation is input to a computational fluid dynamics solver or similar models.

For such measurements to be clinically relevant, they should be as accurate as possible and thereby the segmentation of coronary artery lumen also needs to be as precise as possible. Currently, segmentation of the coronary artery lumen can be constructed by either fully manual annotation, e.g., drawing a contour of the coronary artery lumen manually, or semi-automated methods, i.e., a combination of automated algorithms with human review and maybe editing.

Semi-automated methods typically automatically determine one or more proposals of the contour of the lumen segmentation for an experienced user such as a cardiologist to review. One indicator of the quality of the one or more proposals of the contour is the qualitative fit of the contour of the lumen segmentation to the image data. This can be verified by the experienced user. However, due to various uncertainties including imaging artifacts, segmentation algorithm inaccuracies, etc., a user may disagree with a fully automatic lumen segmentation proposal and thus requires tools for correction or editing.

Standard contour editing tools may comprise dragging individual points or a group of points on the contour with mouse interaction, adding or removing individual points or a group of points, and so on. However, the disagreement of the experienced user may be localized to specific regions, e.g., regions where abnormalities such as stenoses are present or image quality is low. Consequently, such complex user input for generating or manipulating the contour of the lumen segmentation may be incompatible with an interventional setting, e.g., settings of percutaneous coronary intervention (PCI). This is due to inability to use normal editing devices such as a computer mouse or time constraints that do not allow for tedious editing steps.

Further, either fully manual annotation of the contour or semi-automated determination of the contour according to prior art implementations operates on a single cardiac image, e.g., a frame of an X-ray angiogram. Further, either fully manual annotation of the contour or semi-automated determination of the contour according to prior art implementations operates on or relies on a single cardiac image, e.g., a frame of an X-ray angiogram. Even when the single frame is selected by either an experienced user or by an elaborate algorithm and taking factors like the presence and severity of foreshortening, vessel overlaps, image artifacts/quality, panning, vessel out-of-view, and/or sub-optimal contrast agent, the existing methods still cannot construct a segmentation of the coronary artery lumen precisely and reliably because they intrinsically do not consider lumen information from multiple time points of a cardiac cycle (or heartbeat, or heart cycle) in which the lumen diameter varies along the time points of the cardiac cycle.

EP 2 966 615 A2 teaches improving the accuracy of clinical decisions based on non-invasively computed hemodynamic indices where a plurality of candidate locations for each vertex of an anatomical surface model is determined based on uncertainties determined by a trained classifier US 2018/0344173 A1 teaches image-based computational methods and systems for use in estimating a pressure drop and fractional flow reserve (FFR) within a blood vessel where the geometrical difference between actual lumen and the reference lumen diameter is used to characterised stenosis. US 2015/0272448 A1 teaches methods and systems for patient-specific modeling of blood flow where characteristics of a coronary branch geometry include average, minimum, and maximum upstream/downstream cross-sectional areas, or distances along the vessel centerline to the centerline point of minimum and maximum upstream/downstream cross-sectional areas.

### SUMMARY

Therefore, a need exists for advanced techniques for editing contours of coronary arteries. Specifically, a need exists for advanced techniques of finely and precisely adjusting or manipulating a contour of a given lumen segmentation of a portion of the coronary arteries. A need exists for precisely and reliably determining a contour of a given lumen segmentation of a portion of the coronary arteries for a specific patient.

This need is met by the features of the independent claims.

The features of the dependent claims define embodiments.

A computer-implemented method for processing multiple cardiac images is provided. The method comprises obtaining multiple cardiac images. Each of the multiple cardiac images depicts a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, a maximum stenosis severity profile and a minimum stenosis severity profile associated with the portion of the coronary arteries. The method still further comprises determining, based on the maximum stenosis severity profile and the minimum stenosis severity profile, respective magnitudes of one or more local segmentation uncertainties. Each of the one or more local segmentation uncertainties is associated with a respective segment of a contour of a given lumen segmentation of the portion of the coronary arteries.

A computing device comprising a processor and a memory is provided. Upon loading and executing program code from the memory, the processor is configured to perform the computer-implemented method for processing multiple cardiac images.

An angiography device comprising a computing device is provided. The computing device comprises a processor and a memory. Upon loading and executing program code from the memory, the processor is configured to perform the computer-implemented method for processing multiple cardiac images.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates aspects with respect to a C-arm machine.
FIG. 2 schematically illustrates an exemplary 3D frontal view of a heart and of coronary arteries.
FIG. 3 is a flowchart of a method according to various examples.
FIG. 4 schematically illustrates multiple frames of an angiogram acquired at different time points.
FIG. 5 is a flowchart of a further method according to various examples.
FIG. 6 schematically illustrates a zoomed-in portion of an exemplary frame of an angiogram overlaid with segmentation contours and a centerline according to various examples.
FIG. 7 schematically illustrates various exemplary curves respectively depicting different sets of lumen radius measurements according to various examples.
FIG. 8 schematically illustrates various exemplary curves respectively depicting different aligned sets of lumen radius measurements according to various examples.
FIG. 9 schematically illustrates an exemplary maximum stenosis severity profile and an exemplary minimum stenosis severity profile according to various examples.
FIG. 10A and FIG. 10B schematically illustrate an exemplary contour adjustment of a lumen segmentation of a portion of coronary arteries according to various examples.
FIG. 11 schematically illustrates an exemplary machine learning algorithm according to various examples.
FIG. 12 is a block diagram of a computing device according to various examples.

### DETAILED DESCRIPTION OF THE DRAWINGS

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques for adjusting or editing respective segments of a contour of a (given) lumen segmentation of a portion of coronary arteries are described. The (given) lumen segmentation of the portion of the coronary arteries may be determined using any one of the existing methods. Such methods may comprise any one disclosed or referred to in a non-patent literature - Gharleghi, Ramtin, et al. "Towards automated coronary artery segmentation: A systematic review." Computer Methods and Programs in Biomedicine (2022): 107015, or another non-patent literature - Kerkeni, Asma, et al. "A coronary artery segmentation method based on multiscale analysis and region growing." Computerized Medical Imaging and Graphics 48 (2016): 49-61.

According to this disclosure, techniques for processing multiple cardiac images, each of which depicts the same portion of coronary arteries within an anatomical region of interest, are disclosed. For example, each of the multiple cardiac images may depict the same portion of either the left coronary artery or the right coronary artery of the same patient.

The multiple cardiac images may be obtained either during or after an angiography exam of the portion of coronary arteries within the anatomical region of interest. The processing may take place either during or after the angiography exam of the portion of coronary arteries, i.e., either real-time processing or post-processing is possible.

The multiple cardiac images, each of which depicts a portion of coronary arteries, i.e., the same portion of coronary arteries, within an anatomical region of interest, are obtained either during or after an angiography exam. Respective magnitudes of one or more local segmentation uncertainties are determined based on the multiple cardiac images. Each of the one or more local segmentation uncertainties is associated with a respective segment of a contour of a (given) lumen segmentation of the portion of the coronary arteries. The respective segments of the contour are adjusted (edited or manipulated) based on the respective magnitudes of the one or more local segmentation uncertainties.

In general, acquisition of cardiac images is inherently noisy and has limitations related to the achievable resolution as well as artifact, motion, and aliasing errors. Consequently, determination or construction of a contour of a lumen segmentation of a portion of the coronary arteries from cardiac images may be affected by image uncertainty.

For example, the size of the same stenosis may look different from different views and thereby in different frames acquired from different acquisition angles.

The techniques disclosed herein can leverage segmentation uncertainties derived from image processing and lumen segmentation procedures. In particular, inconsistencies and variabilities in lumen radius profiles extracted separately from different frames visualizing the same target vessel at different time points can be exploited to extract quantifiable localized uncertainty measures or magnitudes, which can facilitate smart, precise, and efficient editing, adjusting, or manipulating of a proposed contour of a (given) lumen segmentation of a portion of coronary arteries.

According to various examples, the multiple cardiac images may be acquired at different time points of a cardiac cycle. For example, the multiple cardiac images may be acquired during a cardiac cycle of 1 second using a C-arm X-ray machine with a frame rate of 10. I.e., the multiple cardiac images encapsulate the lumen information of the portion of the coronary arteries from multiple time points of a single cardiac cycle, e.g., changes in respective lumen diameter values at different locations of the portion of the coronary arteries.

By processing the multiple cardiac images using techniques disclosed herein, magnitudes of one or more local segmentation uncertainties associated with respective segments of a contour of a given lumen segmentation of a portion of coronary arteries can be precisely determined, and thereby the respective segments of the contour can be finely and precisely adjusted or edited based on the respective magnitudes of the one or more local segmentation uncertainties. For example, the respective magnitudes of the one or more local segmentation uncertainties can be determined taking lumen radius variability during an entire cardiac cycle into account.

In general, the cardiac cycle is the performance of the human heart from the beginning of one heartbeat to the beginning of the next. It consists of two periods: one during which the heart muscle relaxes and refills with blood, called diastole, following a period of robust contraction and pumping of blood, called systole. After emptying, the heart relaxes and expands to receive another influx of blood returning from the lungs and other systems of the body, before again contracting to pump blood to the lungs and those systems. A normally performing heart must be fully expanded before it can efficiently pump again. Assuming a healthy heart and a typical rate of 70 to 75 beats per minute, each cardiac cycle, or heartbeat, takes about 0.8 second to complete the cycle.

Events and details of cardiac cycles can be illustrated using a Wiggers diagram. In the Wiggers diagram, the X-axis is used to plot time subdivided into the cardiac phases, while the Y-axis typically contains the following on a single grid: blood pressure, aortic pressure, ventricular pressure, atrial pressure, ventricular volume, and electrocardiogram. Accordingly, a specific cardiac cycle can be determined using electrocardiography.

According to various examples, the multiple cardiac images may be obtained directly from an angiography device or from a database for storing the multiple cardiac images acquired by the angiography device, e.g., a picture archiving and communication system (PACS).

Generally, the typical maximum frame rates found on an angiography device such as a C-arm X-ray machine are 8, 15, or 30. This means that during a single cardiac cycle of 0.8 second, 6, 12, or 24 frames can be acquired by a C-arm X-ray machine.

According to various examples, the angiography device may comprise a C-arm X-ray machine, an intravascular ultrasound machine, an optical coherence tomography machine, or a coronary computed tomography angiography machine.

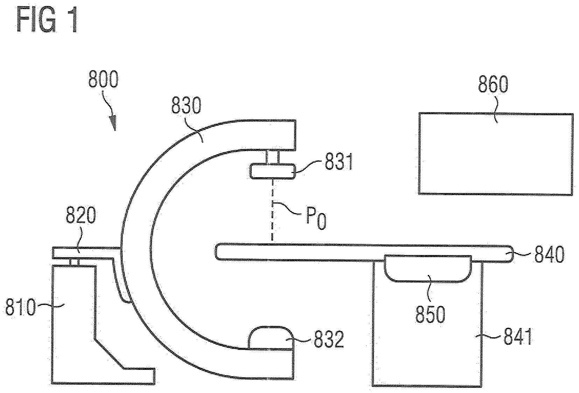

As an exemplary angiography device, FIG. 1 schematically illustrates aspects with respect to a C-arm machine 800. The C-arm machine 800 may be used for both angiography and fluoroscopy. In FIG. 1, the C-arm machine 800 is in neutral position P₀. It is possible to manipulate the C-arm machine 800 to be in a rotated position deviating from the neutral position P₀. The angle between the neutral position P0 and a specific rotated position is referred to as the angiography angle or the fluoroscopy angle. The C-arm machine 800 comprises a rotatable C arm 830 on which X-ray emission means 831 and X-ray detection means 832 may be mounted. The C arm 830 and thereby X-ray emission means 831 and X-ray detection means 832 are positioned to center around patient surface 840. X-ray emission means 831 may emit X-rays which may penetrate through a patient positioned on the patient surface 840. X-ray detection means 832 detects the X -rays emitted from X-ray emission means 831. When a patient on the patient surface 840 is injected with a radio-opaque contrast agent into the patient's vessels, some of the X-rays emitted by X-ray emission means 831 are absorbed by the radio-opaque contrast agent, leading X-ray detection means 832 to detect a sequence of images of the vessels filled with the radio-opaque contrast agent, i.e. an angiogram. X-ray emission means 831 and X-ray detection means 832 may also collectively be referred to as x-ray imaging means.

The C arm 830 may be coupled to a C arm rotation unit 820. The C arm rotation unit 820 may be any motorized means configured to rotate the C arm 830 according to an angiography angel or a fluoroscopy angle. The C arm rotation unit 820 may be attached to and controlled by a C arm control until 810. The C arm control unit 810 may be any kind of circuitry capable of controlling C arm 830. For example, the C arm control unit 810 may include a computing device.

The C-arm machine 800 may further include a control panel 850 mounted onto a side surface of patient surface support 841. The control panel 850 may be used to control C arm 830 in order to guide medical specialists to pathological vessels. Fig. 1 does not show any connections between control panel 850 and C arm 830 to simplify the depiction of the exemplary C-arm machine 800. In some examples, the connection may be wireless. In some further examples, the connection may be wired and may e.g., be integrated into the ceiling of the room where the C-arm machine 800 is located.

The C-arm machine 800 may also include a display 860. The display 860 may be used to display information to the medical specialist, such as real-time fluoroscopy image with an overlaid vessel roadmap image including a path to one or more pathological vessels, and/or a location of the pressure wire for measuring the at least one measurement associated with the at least one hemodynamic index. Further, the display 860 may be used to display vessel segmentation data included in overlaid vessel roadmap images, including labels for various vessel segments. In some examples, display 860 may be a touch screen, which may be used to toggle the display of the vessel segmentation data on and off.

According to this disclosure, the display 860 may be used to display a contour of a given lumen segmentation of a portion of coronary arteries and provide to a user with adjusting or editing tools for adjusting or editing one or more segments of the contour. Optionally, the display 860 may visualize the respective magnitudes of the one or more local segmentation uncertainties together with the contour, and the user can adjust or edit the respective segments of the contour based on the respective magnitudes of the one or more local segmentation uncertainties, e.g., by touching and/or dragging on the display.

The C-arm machine 800 may be connectable to a database (not shown in FIG. 1), such as a PACS located within a local network of a hospital, for storing angiograms, and/or contours of lumen segmentation of various portions of coronary arteries, and/or respective magnitudes of segmentation uncertainties associated with the contours.

According to various examples, a name (e.g., any one in the column "Abbreviation" in Table 1) of a specific segment of a coronary artery or of the entire coronary artery can be determined based on an anatomical region of interest of the angiography exam. For example, such an anatomical region of interest may comprise the left or right branch of the coronary vessel tree or a specific vessel section. The anatomical region of interest may be determined based on user input, or on a patient's electronic medical record included in a health information system. Additionally or optionally, the anatomical region of interest may be determined based on multiple predefined seed points.

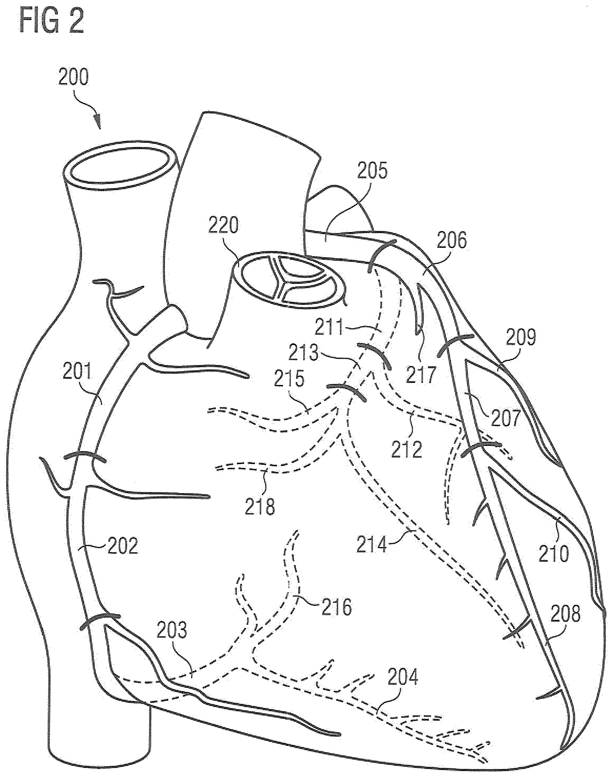

For example, the portion of coronary arteries within the anatomical region of interest may be one or more segments of the coronary arteries of the heart. FIG. 2 schematically illustrates an exemplary 3D frontal view 200 of a heart and of coronary arteries. The segments of the coronary arteries in FIG. 2 are based on and numbered according to the vessel segmentation as proposed by the American Heart Association (AHA) and as amended by the Society of Cardiovascular Computed Tomography (SCCT). Table 1 provides a short description of the respective vessel segments as well as an abbreviation for each vessel segment.

**Table 1: Segments of the Coronary Arteries in FIG. 2.**

| **Ref . Sign** | **Vessel Segment** | **Abbreviation** | **Description** |
|---|---|---|---|
| 201 | proximal right coronary artery | pRCA | Ostium of the RCA to one-half the distance to the acute margin of heart |
| 202 | Mid RCA | mRCA | End of pRCA to the acute margin of heart |
| 203 | Distal RCA | dRCA | End of mRCA to origin of the PDA (posterior descending artery) |
| 204 | PDA-R | R-PDA | PDA from RCA |
| 205 | Left main | LM | Ostium of LM to bifurcation of LAD (left anterior descending artery) and |
| | | | LCx (left circumflex artery) |
| 206 | Proximal LAD | pLAD | End of LM to the first large septal or D1 (first diagonal), whichever is most proximal |
| 207 | Mid LAD | mLAD | End of proximal LAD to one-half the distance to the apex |
| 208 | Distal LAD | dLAD | End of mid LAD to end of LAD |
| 209 | D1 | D1 | First diagonal branch D1 |
| 210 | D2 | D2 | Second diagonal branch D2 |
| 211 | Proximal LCx | pCx | End of LM to the origin of the OM1 (first obtuse marginal) |
| 212 | OM1 | OM1 | First OM1 traversing the lateral wall of the left ventricle |
| 213 | Mid and distal LCx | LCx | Traveling in the atrioventricular groove, distal to the OM1 branch to the end of the vessel or origin of the L-PDA |
| 214 | OM2 | OM2 | Second marginal OM2 |
| 215 | PDA-L | L-PDA | PDA from LCx |
| 216 | PLB-R | R-PLB | PLB from RCA |
| 217 | Ramus intermedius | RI | Vessel originating from the left main between the LAD and LCx in case of a trifurcation |
| 218 | PLB-L | L-PLB | PLB from LCx |

In addition to the vessel segments listed in Table 1, FIG. 2 also indicates the aortic valve 220.

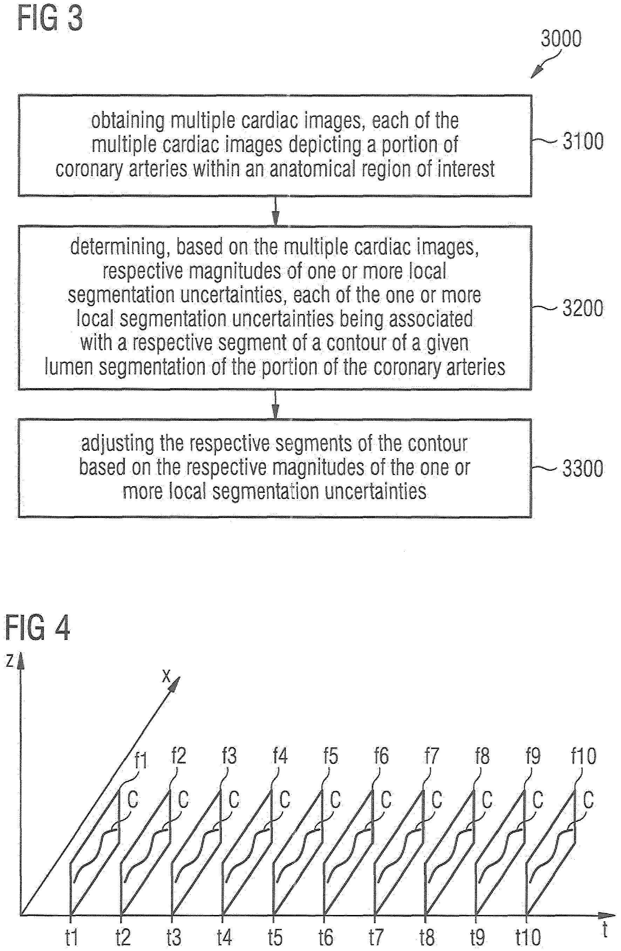

FIG. 3 illustrates aspects with respect to processing multiple cardiac images, each of which depicts a portion of coronary arteries, i.e., the same portion of coronary arteries, e.g., LAD (i.e., a combination of pLAD 206, mLAD 207, and dLAD 208), or RCA (i.e., a combination of pRCA 201, mRCA 202, and dRCA 203) as shown in FIG. 2, within an anatomical region of interest. The multiple cardiac images are obtained either during or after an angiography exam and thereby the processing can be either real-time processing or post-processing. Respective magnitudes of one or more local segmentation uncertainties are determined based on the multiple cardiac images. Each of the one or more local segmentation uncertainties is associated with a respective segment of a contour of a (given) lumen segmentation of the portion of the coronary arteries. The respective segments of the contour are adjusted (edited or manipulated) based on the respective magnitudes of the one or more local segmentation uncertainties. Details of the method 3000 are described below.

Block 3100: obtaining multiple cardiac images, each of the multiple cardiac images depicting a portion of coronary arteries within an anatomical region of interest.

According to various examples, the multiple cardiac images may comprise cardiac images acquired under different acquisition angles.

For example, the multiple cardiac images may be obtained directly from an angiography device, e.g., the C-arm machine 800 of FIG. 1, under different acquisition angles. I.e., the multiple cardiac images may respectively depict different views of the same portion of coronary arteries to increase accuracy and robustness of the determination of respective magnitudes of one or more local segmentation uncertainties and/or radius measurements of the portion of the coronary arteries. For example, the portion of the coronary arteries may comprise one or more stenoses and the true severity of an individual stenosis may need to be revealed by looking at it from multiple views, and multiple views can also help solve foreshortening-related issues.

Alternatively, the multiple cardiac images may be obtained from a database for storing the multiple cardiac images acquired by the angiography device, e.g., a PACS.

According to various examples, the multiple cardiac images may comprise cardiac images acquired using multi-phase coronary computed tomography angiography. I.e., the multiple cardiac images may be obtained from imaging data acquired using computed tomography angiography.

Hereinafter, techniques will be explained using frames of X-ray angiograms as an example of cardiac images. I.e., a single cardiac image may correspond to a frame of an angiogram.

FIG. 4 schematically illustrates multiple frames f1-f10 of an angiogram acquired at different time points t1-t10 and each of the multiple frames f1-f10 depicts the same portion C of the coronary arteries within an anatomical region of interest.

For example, the multiple frames f1-f10 may be acquired during a single cardiac cycle of 1 second using a C-arm X-ray machine with a frame rate of 10. I.e., the multiple frames f1-f10 encapsulate the lumen information of the portion C of the coronary arteries from multiple time points of a single cardiac cycle, e.g., changes in respective lumen diameter or radius values at different locations of the portion C of the coronary arteries.

To improve the accuracy of determination of the respective magnitudes of one or more local segmentation uncertainties associated with respective segments of a contour of a given lumen segmentation of the portion C of the coronary arteries, it is possible to automatically and precisely select or determine, among frames of an angiogram, multiple appropriate frames, e.g., the multiple frames f1-f10, where the entire portion C of the coronary arteries is visible.

According to various examples, said obtaining of the multiple cardiac images may comprise obtaining an angiogram acquired during an angiography exam of the anatomical region of interest and selecting, based on at least one pre-defined criterion, the multiple cardiac images among frames of the angiogram.

Such pre-defined criterion may comprise at least one of the following:
- the portion of coronary arteries is well visible;
- minimal foreshortening appears;
- no or minimal vessel overlaps appear;
- image quality, especially around the lumen borders, is good, e.g., above a threshold;
- no artifact presents; and
- the entire portion of coronary arteries is in the field of view.

For example, the multiple cardiac images may be selected from frames acquired in a single cardiac cycle, e.g., selecting 5 frames from the 10 frames f1-f10 acquired in a single cardiac cycle.

Alternatively or optionally, the multiple cardiac images may be selected from frames acquired in different cardiac cycles. For example, an acquired angiogram may comprise 100 frames acquired in 10 cardiac cycles, e.g., first to tenth cardiac cycles, and 10 frames, e.g., first to tenth frames, are acquired in each cardiac cycle. A first cardiac image may be selected from respective first frames acquired in respective cardiac cycles. A further or second cardiac image may be selected from respective second (or any one of the third to tenth) frames acquired in respective cardiac cycles.

As a further example, 10 cardiac images may be selected from frames acquired in different cardiac cycles. Each one of the 10 cardiac images may be selected from respective frames acquired at the same time point within respective cardiac cycles. E.g., the first cardiac image, e.g., f1, among the 10 cardiac images may be selected from all the first frames respectively acquired in the first to tenth cardiac cycles; the second cardiac image, e.g., f2, among the 10 cardiac images may be selected from all the second frames respectively acquired in the first to tenth cardiac cycles; and so on.

According to various examples, after obtaining the multiple cardiac images, the method 3000 may further comprise determining the anatomical region of interest based on multiple predefined seed points.

For example, proximal seed points may comprise the catheter tip, and distal seed points may comprise one or more points selected on the main vessels or on side branches with significant stenoses.

Optionally, the portion of coronary arteries may be determined based on angulation information for acquiring the multiple cardiac images.

Optionally or additionally, the method 3000 may further comprise registering the multiple cardiac images.

For example, the proximal and distal seed points may be co-registered across the multiple cardiac images to ensure that they represent the same anatomical region of interest.

According to various examples, said registration or co-registration may comprise applying a diffeomorphic registration algorithm, e.g., as disclosed in a non-patent literature - Ashburner, John. "A fast diffeomorphic image registration algorithm." Neuroimage 38.1 (2007): 95-113., on the vesselness maps extracted from each frame; using the obtained deformation fields to track each of the seed points from each frame onto all of the other frames; and aggregating the tracked seed points via a density-based clustering algorithm such as DB Scan (i.e., Density-based spatial clustering of applications with noise), e.g., as disclosed in a non-patent literature - Hu, Lihua, et al. "KR-DBSCAN: A density-based clustering algorithm based on reverse nearest neighbor and influence space." Expert Systems with Applications 186 (2021): 115763.

According to various examples, the method 3000 may further comprise segmenting the anatomical region of interest across the multiple cardiac images, i.e., separating the anatomical region of interest from the background in each of the multiple cardiac images.

Block 3200: determining, based on the multiple cardiac images, respective magnitudes of one or more local segmentation uncertainties, each of the one or more local segmentation uncertainties being associated with a respective segment of a contour of a given lumen segmentation of the portion of the coronary arteries.

According to various examples, the respective magnitudes of the one or more local segmentation uncertainties may be determined based on variabilities in respective lumen radii of the portion of the coronary arteries, e.g., variabilities in lumen radii respectively determined based on the multiple cardiac images.

For example, the respective magnitudes of the one or more local segmentation uncertainties may be determined based on variabilities in respective lumen radii of the portion C of the coronary arteries as shown in FIG. 4, e.g., variabilities in lumen radii respectively determined based on the ten frames f1-f10.

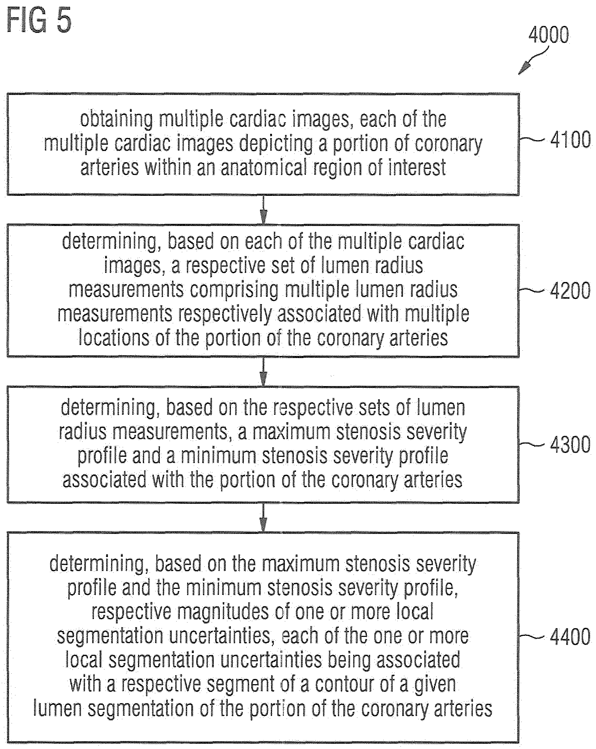

According to various examples, the respective magnitudes of the one or more local segmentation uncertainties may be determined using the method 4000 as shown in FIG. 5. The method 4000 will be described below.

FIG. 5 illustrates aspects with respect to processing multiple cardiac images, each of which depicts a portion of coronary arteries, i.e., the same portion of coronary arteries, e.g., LAD (i.e., a combination of pLAD 206, mLAD 207, and dLAD 208), or RCA (i.e., a combination of pRCA 201, mRCA 202, and dRCA 203) as shown in FIG. 2, within an anatomical region of interest. The multiple cardiac images are obtained either during or after an angiography exam and thereby the processing can be either real-time processing or post-processing. A respective set of lumen radius measurements is determined based on each of the multiple cardiac images and comprises multiple lumen radius measurements respectively associated with multiple locations of the portion of the coronary arteries. A maximum stenosis severity profile and a minimum stenosis severity profile associated with the portion of the coronary arteries are respectively determined based on the respective sets of lumen radius measurements. Respective magnitudes of one or more local segmentation uncertainties are determined based on the maximum stenosis severity profile and the minimum stenosis severity profile. Each of the one or more local segmentation uncertainties is associated with a respective segment of a contour of a given lumen segmentation of the portion of the coronary arteries. Details of the method 4000 are described below.

Block 4100: obtaining multiple cardiac images, each of the multiple cardiac images depicting a portion of coronary arteries within an anatomical region of interest.

According to this disclosure, block 4100 may correspond to block 3100 of the method 3000 as described above and shown in FIG. 3.

Block 4200: determining, based on each of the multiple cardiac images, a respective set of lumen radius measurements comprising multiple lumen radius measurements respectively associated with multiple locations of the portion of the coronary arteries.

According to various examples, the lumen radius measurements may be determined or calculated using any one of the existing techniques or algorithms related to quantitative coronary angiography. For example, non-patent literature - Garrone, Paolo, et al. "Quantitative coronary angiography in the current era: principles and applications." Journal of interventional cardiology 22.6 (2009): 527-536. - provides a comprehensive and updated viewpoint on quantitative coronary angiography. It is also conceivable that various existing machine learning algorithms or image processing techniques can be used to determine the lumen radius measurements.

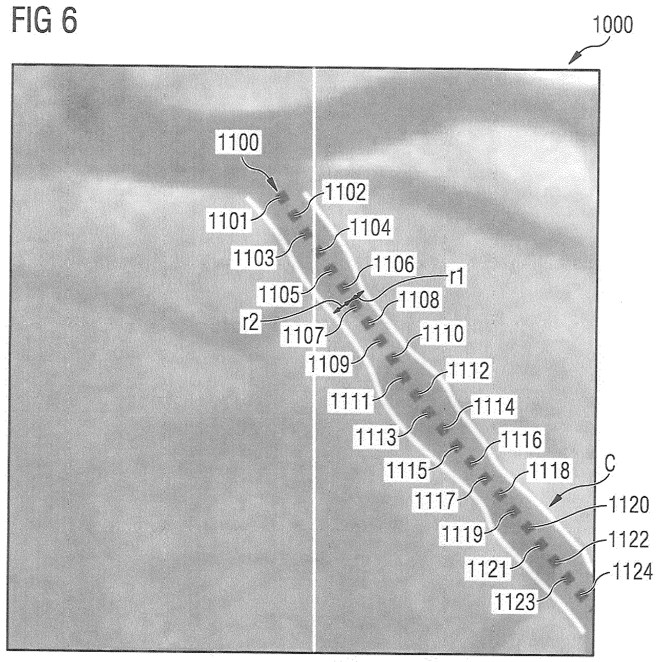

FIG. 6 schematically illustrates one exemplary frame 1000 of an angiogram according to various examples. Specifically, FIG. 6 schematically illustrates a zoomed-in portion of an exemplary frame of an angiogram overlaid with segmentation contours and a "centerline" (dashed line). The frame 1000 may depict the portion C of coronary arteries as shown in FIG. 4. A centerline 1100 is generated between the starting point 1101 and the ending point 1124 of the portion C of coronary arteries. Between the starting point 1101 and the ending point 1124, there are multiple location points 1102-1123. For each of the location points 1101-1124, a respective lumen radius associated with such a location point may be measured or determined, and thereby a set of lumen radius measurements comprising the respective lumen radius at each of the location points 1101-1124 can be determined or calculated.

For example, at location point 1107, two lumen radii r1 and r2 may be determined or measured. The respective lumen radius associated with location point 1107 may be determined as an average of r1 and r2. It is also possible to determine or select the lumen radius associated with location point 1107 as either r1 or r2. Optionally, r1 and r2 may be determined based on different frames acquired using different acquisition angles.

According to various examples, for each of the location points 1101-1124, it is also possible to determine multiple, e.g., 3, 4, 8, or even more, lumen radii based on multiple frames acquired using different acquisition angles, and determine an average of the multiple lumen radii as the respective lumen radius for the respective set of lumen radius measurements.

Similarly, for each one of the multiple frames f1-f10 of FIG. 4, a respective set of lumen radius measurements comprising the respective lumen radius at each of the location points 1101-1124 can be determined or calculated. I.e., ten sets s1-s10 of lumen radius measurements can be determined respectively for the ten frames f1-f10, and each of the ten sets s1-s10 comprises lumen radius measurements respectively associated with the location points 1101-1124.

To facilitate the determination of the respective sets s1-s10 of the lumen radius measurements, a segmentation algorithm may be applied to the ten frames f1-f10, respectively, to separate the portion C of the coronary arteries from the background of the respective frame.

According to various examples, said determining of the respective set of lumen radius measurements may comprise segmenting the portion C of the coronary arteries from the respective cardiac image of the multiple cardiac images f1-f10 and determining the respective set s1-s10 of the lumen radius measurements based on the respective segmented portion of the coronary arteries.

According to various examples, after segmenting the portion C of the coronary arteries from the respective cardiac image of the multiple cardiac images f1-f10, the respective segmented portion of the coronary arteries may be co-registered along the centerline 1100 and/or based on landmarks detected in the respective frames f1-f10, e.g., bifurcations, stenoses, calcifications, and etc.

Block 4300: determining, based on the respective sets of lumen radius measurements, a maximum stenosis severity profile and a minimum stenosis severity profile associated with the portion of the coronary arteries.

According to various examples, the maximum stenosis severity profile and the minimum stenosis severity profile may be respectively determined based on local concavities and/or local convexities of multiple curves and each of the multiple curves may depict a respective relationship between a respective set of the lumen radius measurements and the multiple locations of the portion of the coronary arteries.

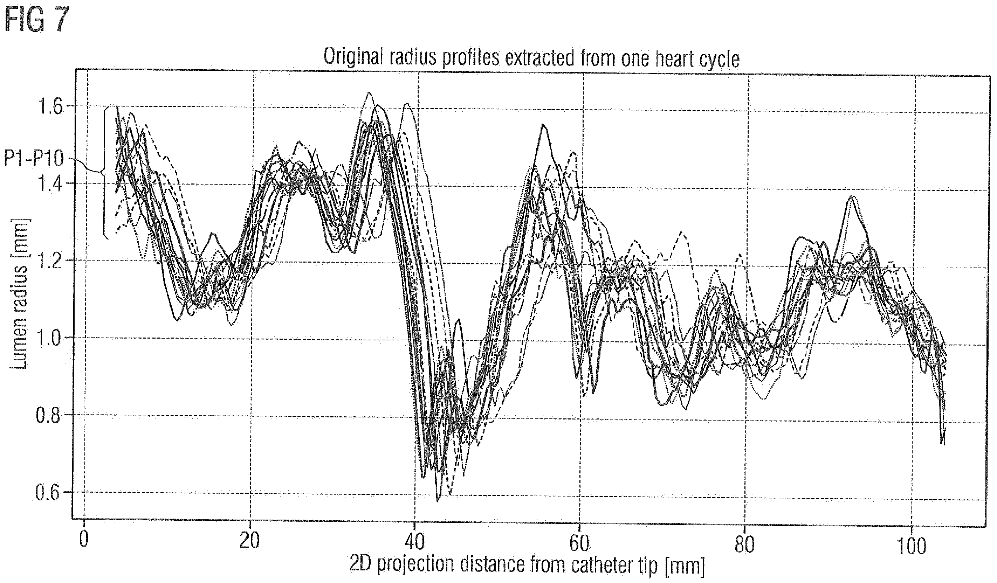

For example, as shown in FIG. 7, after determining the respective sets s1-s10 of the lumen radius measurements, respective curves (or original radius profiles) p1-p10 may be determined or plotted for each of the sets s1-s10 using interpolation such as linear interpolation, polynomial interpolation, spline interpolation, and/or mimetic interpolation. Optionally, the sets s1-s10 may be determined based on frames f1-f10 acquired in a single cardiac cycle or heart cycle. The x-axis of each curve may indicate respective distances (or 2D projection distances) between the catheter tip and each of the location points 1101-1124, and the y-axis may indicate respective lumen radii associated with the respective location points. Then, the maximum stenosis severity profile and the minimum stenosis severity profile may be respectively determined based on local concavities or convexities of the respective curves p1-p10. For example, the second derivatives at each of the location points 1101-1124 may be calculated for each of the respective curves p1-p10.

At a location point of a curve, if the second derivative is positive the curve is concave up (also referred to as convex) at such a location point, meaning that the tangent line at such a location point lies below the curve; similarly, if the second derivative is negative the curve is concave down (also simply called concave) at such a location point, meaning that the tangent line at such a location point lies above the curve; if the second derivative changes sign, the curve will switch from concave down to concave up, or vice versa, and a point where this occurs is called an inflection point.

According to various examples, the second derivatives may be computed via central finite differences.

In practice, due to vessel deformations caused by heart motion, the respective sets s1-s10 of the lumen radius measurements (or the radius profiles/curves p1-p10) may be not aligned. To more precisely determine the respective values of the at least one hemodynamic indices, the respective sets s1-s10 of the lumen radius measurements (or the radius profiles/curves p1-p10) may be warped before determining the maximum stenosis severity profile and the minimum stenosis severity profile.

For example, a dynamic time warping algorithm, e.g., as disclosed in a non-patent literature - Salvador, Stan, and Philip Chan. "Toward accurate dynamic time warping in linear time and space." Intelligent Data Analysis 11.5 (2007): 561-580. - may be used for warping the respective sets s1-s10 of the lumen radius measurements. Alternatively, it is also possible to use an optimal subsequence bijection algorithm such as that disclosed in a non-patent literature - Latecki, Longin Jan, et al. "Optimal subsequence bijection." Seventh IEEE International Conference on Data Mining (ICDM 2007). IEEE, 2007.

Optionally or additionally, said determining of the maximum stenosis severity profile and the minimum stenosis severity profile may comprise determining a reference set of the lumen radius measurements, aligning each of the respective sets of the lumen radius measurements with the reference set of the lumen radius measurements, and determining the maximum stenosis severity profile and the minimum stenosis severity profile based on the respective aligned sets of the lumen radius measurements.

For example, the reference set of the lumen radius measurements may be randomly or arbitrarily selected from the ten sets s1-s10 of the lumen radius measurements. For instance, s1 is selected as the reference set of the lumen radius measurements and the other sets s2-s10 can be respectively aligned with the reference set s1 using dynamic time warping algorithm, e.g., the dtw function of Matlab, i.e., executing dtw (s1,s2), dtw (s1,s3), dtw (s1,s4), ..., dtw (s1,s10).

According to various examples, said determining of the reference set of the lumen radius measurements may comprise selecting the set of the lumen radius measurements having the longest length as the reference set of the lumen radius measurements. Optionally or additionally, the set of the lumen radius measurements having the longest length may be determined by sorting the respective sets of the lumen radius measurements based on respective lengths of the respective sets in descending or ascending order.

For example, each of the ten sets s1-s10 of the lumen radius measurements may be indicated as a row or column vector, and the length of a respective set corresponds to the number of elements or entries of the respective vector.

According to various examples, said aligning of each of the respective sets of the lumen radius measurements with the reference set of the lumen radius measurements may comprise, for each of the respective unaligned sets of the lumen radius measurements, iteratively performing the following steps:
- selecting the longest set of the lumen radius measurements among all the unaligned sets of the lumen radius measurements;
- aligning the selected set of the lumen radius measurements with the reference set of the lumen radius measurements; and
- updating the reference set of the lumen radius measurements based on all aligned sets of the lumen radius measurements.

For example, after sorting the respective sets s1-s10 of the lumen radius measurements based on respective lengths of the respective sets s1-s10 in descending order, the first sorted set, i.e., the longest set, e.g., s5, may be selected as the initial reference set and then the second longest set, e.g., s2, may be aligned with the initial reference set s5 to obtain the aligned set s2' of s2. Next, the reference set is updated based on the initial reference set s5 and the aligned set s2' of s2. For example, the reference set may be determined or calculated based on either an element-wise average or an element-wise weighted average of s5 and s2'. Similarly, the third longest set may be aligned with the updated reference set and then the reference set may be updated again by further considering the aligned set of the third longest set. I.e., said aligning and updating may be iteratively performed until the shortest set has been aligned.

Optionally or additionally, said aligning may be based on one or more anatomical landmarks within the anatomical region of interest.

For example, the one or more anatomical landmarks may be associated with the portion of coronary arteries, e.g., bifurcations, stenoses, calcifications, and etc.

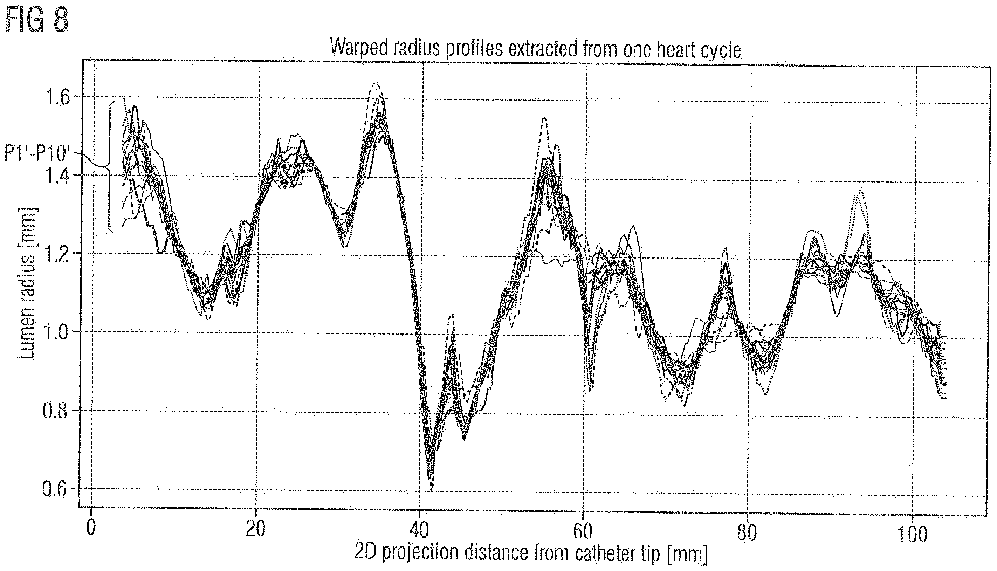

FIG. 8 schematically illustrates various exemplary curves respectively depicting different aligned sets of lumen radius measurements according to various examples. For example, FIG. 8 may schematically illustrate respective aligned/warped curves p1'-p10' of the various exemplary curves p1-p10 shown in FIG. 7. I.e., the respective aligned curves p1'-p10' shown in FIG. 8 can be determined or plotted for each of the aligned sets s1'-s10' of s1-s10 using interpolation. The curves p1'-p10' shown in FIG. 8 may comprise warped radius profiles extracted from one cardiac/heart cycle.

According to various examples, the maximum stenosis severity profile and the minimum stenosis severity profile may be respectively determined based on local concavities and/or local convexities of the multiple aligned curves p1'-p10'.

In general, coronary stenosis refers to an abnormally narrowed coronary artery. The stenosis is usually discrete, but may sometimes be diffuse and involve a long segment of an artery. In coronaries, stenoses are usually located in the large epicardial arteries with diameters of more than 2 mm, but they may also occur in smaller arteries. In the majority of cases, a stenosis is caused by atherosclerosis, but other causes such as systemic inflammatory diseases may be involved. Coronary stenoses are usually chronic fixed changes, but sometimes a transient, dynamic stenosis due to spasm of the artery may happen.

From a pathophysiological point of view, a coronary stenosis impairs flow to the subtending myocardium, as the perfusion pressure is decreased distal to the stenosis. The flow dynamics in a vessel is governed by Poiseuille's law, which states that resistance in a vessel has an inverse relationship with the radius of the vessel to the fourth power. For example, the diameter (and therefore the radius as well) of a coronary artery may be reduced by two-thirds (for example, an artery with a diameter of 3 mm with a fixed stenosis at a discrete segment may have a residual diameter of only 1 mm in that segment). In that case, the remaining diameter (and the radius) in the diseased segment will be one-third of the initial diameter/radius, and according to Poiseuille's law, the resistance within that stenotic segment will increase by 81-fold. If the perfusion pressure in that coronary artery remained constant, then the blood flow distal to this stenosis would decrease by 81-fold.

Based on these intrinsic characteristics of coronary artery stenoses, individual stenosis can be determined by a significant reduction followed by a significant increase in the lumen radius value or profiles. An individual stenosis generally starts at a location with a (local) maximum radius and ends at a location with a further (local) maximum radius. Between the two local maximum radius locations, there is a significant reduction in the radius value, i.e., the segment of the lumen radius profile between the two local maximum radius locations looks like a convex curve.

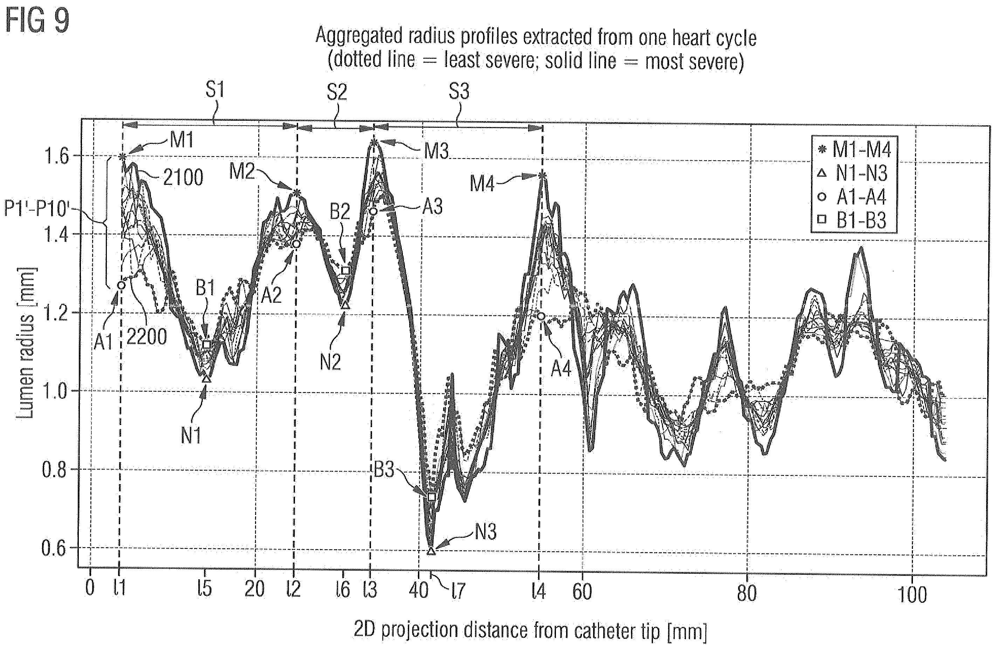

FIG. 9 schematically illustrates an exemplary maximum stenosis severity profile 2100 and an exemplary minimum stenosis severity profile 2200 according to various examples. The maximum stenosis severity profile 2100 and the minimum severity profile 2200 may be determined according to the following steps:
Step 1: determining, based on the aligned curves p1'-p10', one or more local maximum lumen radius of the portion C of the coronary arteries and respective locations of the portion C of the coronary arteries with which the one or more local maximum lumen radius are associated; and determining, based on the aligned curves p1'-p10', respective minimum lumen radii associated with the respective locations with which the one or more local maximum lumen radius are associated.

For example, points M1, M2, M3, and M4 respectively indicate four local maximum lumen radii. The points M1, M2, M3, and M4 are respectively associated with locations 11, 12, 13, and 14 of the portion C of the coronary arteries. After determining the locations 11, 12, 13, and 14, the respective minimum lumen radii associated with the four locations 11, 12, 13, and 14 can be determined, i.e., the respective radii indicated by points A1, A2, A3, and A4.

According to this disclosure, the maximum and minimum lumen radii associated with the same location of the portion C of the coronary arteries may be determined among different frames, e.g., f1-f10, of an angiogram.

Step 2: determining stenoses based on the local maximum lumen radii.

As described above, an individual stenosis generally starts at a location with a (local) maximum radius and ends at a location with a further (local) maximum radius. As shown in FIG. 9, the three segments of curves, e.g., M1M2, M2M3, and M3M4, in between respective two neighboring points of the four points M1, M2, M3, and M4 indicate three stenoses S1 (i.e., between locations 11 and 12), S2 (i.e., between locations 12 and 13), and S3 (i.e., between locations 13 and 14), respectively.

Step 3: determining, based on the aligned curves p1'-p10', a respective local minimum lumen radius for each of the determined stenoses, e.g., S1, S2 and S3, and respective locations of the portion C of the coronary arteries with which the respective local minimum lumen radii are associated; and determining, based on the aligned curves p1'-p10', respective maximum lumen radii associated with the respective locations with which the respective local minimum lumen radii are associated.

As shown in FIG. 9, points N1, N2, and N3 respectively indicate the minimum lumen radii of the three stenoses S1, S2, and S3. The points N1, N2, and N3 are respectively associated with locations 15, 16, and 17 of the portion C of the coronary arteries. After determining the locations 15, 16, and 17, the respective maximum lumen radii associated with the three locations 15, 16, and 17 can be determined, i.e., the respective radii indicated by points B1, B2, and B3.

According to this disclosure, the maximum and minimum lumen radii associated with the same location of the portion C of the coronary arteries may be determined among different frames, e.g., f1-f10, of an angiogram.

Step 4: for each of the determined stenoses, e.g., S1, S2, and S3, determining respective segments of both the maximum severity profile 2100 and the minimum severity profile by interpolating lumen radius values segment-wise from the start to the end of the respective stenosis.

In general, for a specific stenosis such as any one of S1, S2, and S3, the respective segment of the maximum severity profile 2100 has, compared to the respective segment of the minimum severity profile 2200, a larger lumen radius at the start and end of the stenosis, respectively, and on the other hand, has a smaller radius at the location of the minimal lumen radius. This means that the maximum and minimum severity profiles 2100 and 2200 typically intersect with each other within one stenosis.

For example, for stenosis S1, the respective segment of the maximum severity profile 2100 may start from point M1, via point N1, and end at point M2 and the respective segment of the minimum severity profile 2200 may start from point A1, via point B1, and end at point A2; for stenosis S2, the respective segment of the maximum severity profile 2100 may start from point M2, via point N2, and end at point M3 and the respective segment of the minimum severity profile 2200 may start from point A2, via point B2, and end at point A3; for stenosis S3, the respective segment of the maximum severity profile 2100 may start from point M3, via point N3, and end at point M4 and the respective segment of the minimum severity profile 2200 may start from point A3, via point B3, and end at point A4.

According to various examples, for an individual stenosis determined in step 2 explained above, multiple segments of the stenosis may be determined based on one or more local maximum lumen radii between the start and end of the stenosis. For each segment of an individual stenosis, respective segments of the maximum and minimum severity profiles 2100 and 2200 can be determined or revised more precisely by performing the steps 1-4.

Additionally, or optionally, each segment of an individual stenosis may be divided into multiple sub-segments based on one or more local maximum lumen radii between the start and end of the respective segment. For each sub-segment, the maximum and minimum severity profiles 2100 and 2200 can be determined or revised more precisely by performing the steps 1-4, too.

In summary, steps 1-4 can be applied to different segments of the portion C of the coronary arteries and thereby the maximum and minimum severity profiles 2100 and 2200 can be determined. The more segments the more precise the two profiles 2100 and 2200. The techniques disclosed herein can ensure that different local severity values are obtained for each stenosis, while at the same time, the radius profile displays no discontinuities.

Block 4400: determining, based on the maximum stenosis severity profile and the minimum stenosis severity profile, respective magnitudes of one or more local segmentation uncertainties, each of the one or more local segmentation uncertainties being associated with a respective segment of a contour of a given lumen segmentation of the portion of the coronary arteries.

According to various examples, for each location of the portion C of the coronary arteries, e.g., any one of location points 1101-1124 of FIG. 5, a respective magnitude of a respective local segmentation uncertainty associated with a respective location can be determined based on a respective size of the gap between the maximum stenosis severity profile and the minimum stenosis severity profile at the respective location.

For example, referring to FIG. 9, the magnitude of the local segmentation uncertainty associated with location 11 can be determined based on the size of the gap between point M1 and point A1, i.e., M1A1. The size of the gap between point M1 and point A1 may indicate the maximum magnitude of the local segmentation uncertainty associated with location 11, and one or more instances of such a magnitude may be proportional to the size of the gap, e.g., 10%-95% of the size of the gap.

Similarly, the respective magnitudes of the respective local segmentation uncertainties associated with any other locations of the portion C of the coronary arteries, e.g., locations 12-17, can be determined as well.

For an individual segment of the portion C of the coronary arteries, e.g., 11-15, 15-12, 15-16, 12-14, and so on, the respective magnitude of the local segmentation uncertainty associated with the individual segment can be determined based on respective magnitude associated with respective locations of multiple or even all locations of the individual segment. It is also possible to use a magnitude associated with a single location of the individual segment to represent the magnitude associated with the individual segment.

For instance, multiple, e.g., 5-20, location points can be selected or sampled for segment 11-15, and the magnitude associated with the segment 12-15 can be determined by averaging respective magnitudes associated with the multiple location points. Alternatively, it is possible to determine the magnitude associated with the segment 11-15 based on either a weighted sum or a weighted average of the respective magnitudes associated with the multiple location points.

Referring back to FIG. 3, after determining the respective magnitudes of one or more local segmentation uncertainties at block 3200, e.g., using the method 4000 shown in FIG. 5, the method 3000 may further comprise:
Block 3300: adjusting the respective segments of the contour based on the respective magnitudes of the one or more local segmentation uncertainties.

According to this disclosure, the (given) lumen segmentation of the portion of the coronary arteries may be determined using any one of the existing methods. For example, an existing machine-learning-based method may be applied to one or more of the multiple cardiac images obtained at block 3100 to determine or generate the contour of the (given) lumen segmentation of the portion of the coronary arteries.

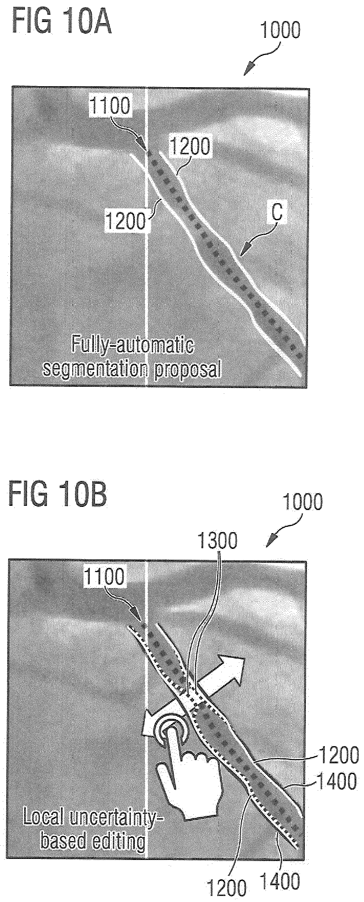

FIG. 10A and FIG. 10B schematically illustrate an exemplary contour adjustment of a lumen segmentation of a portion C of coronary arteries according to various examples. For example, FIG. 10A and FIG. 10B may be together displayed using the display 860 of the C-arm machine 800 of FIG. 1.

As shown in FIG. 10A, a contour 1200 of a lumen segmentation of the portion C of the coronary arteries is visualized in the frame 1000 of FIG. 6. The contour 1200 may be fully automatically determined or generated using an existing segmentation algorithm. A centerline 1100 may be also determined or generated using the existing segmentation algorithm.

FIG. 10B schematically illustrates possible user interaction for adjusting the contour 1200 based on respective magnitudes of one or more local segmentation uncertainties associated with respective segments of the contour 1200.

For example, during a PCI, a cardiologist may review the contour 1200 displayed on the screen of the display 860 of the C-arm machine 800 of FIG. 1. If the cardiologist is not satisfied with the contour 1200, the cardiologist may activate adjusting or editing functionalities for adjusting respective segments of the contour 1200. Then, the contour 1200 may be adjusted by manipulating, adjusting, or setting respective lumen radii (or diameters) at respective location points of the portion C of the coronary arteries. For example, the respective lumen radii (or diameters) can be adjusted or set based on the respective magnitudes of respective local segmentation uncertainties associated with respective location points or segments.

According to various examples, as shown in FIG. 10B, two additional contours 1300 and 1400 respectively corresponding to the maximum stenosis severity profile 2100 and the minimum stenosis severity profile 2200 of FIG. 9 can be displayed together with the contour 1200 to a user such as a cardiologist for assisting adjustment or editing of the contour 1200 based on segmentation uncertainties.

For example, as shown in FIG. 10B, the contour 1200 can be adjusted or edited by leveraging respective magnitudes or levels of local segmentation uncertainties. For example, at the segment indicated by the double-headed arrow, the magnitude of local segmentation uncertainties is high as the contours 1300 and 1400 diverge significantly. The user's interaction for adjusting or editing the contour 1200 at the segment indicated by the double-headed arrow can be implemented as follows:
The user touches or clicks a point on the centerline 1100, then drags to the left/right or orthogonal to the centerline 1100 to adjust the radius at this location and pull or push respective points of the contour 1200 corresponding to this location in the orthogonal direction to the centerline. When underlying uncertainty at this location is high, this could automatically enable larger adjustment steps or less constrained adjustment, and finer steps or more constrained adjustment for lower uncertainty regions. The editing or adjusting could be localized to contours corresponding to that particular touch or click point or extended to edit contour points corresponding to neighboring points on the centerline 1100. In the latter case, the magnitude of adjustments for neighboring points could consider the uncertainty at each point and/or weighted by a factor corresponding to the inverse distance to the original touch or click point.

According to various examples, said adjusting of the respective segments of the contour may comprise for each one of the one or more local segmentation uncertainties, determining whether the respective magnitude is greater than a predefined threshold and upon determining the respective magnitude is greater than the predefined threshold, adjusting the respective segment of the contour.

According to various examples, said adjusting of the respective segments of the contour may be further based on a specific use case (or subsequent processing) of the given lumen segmentation and /or an anatomical structure of the portion of the coronary arteries.

For example, specific use cases may comprise computation of at least one hemodynamic index such as FFR, Quantitative Coronary Analysis (QCA), and PCI planning.

According to various examples, respective magnitudes of local segmentation uncertainties may be weighted given a main scope of the lumen segmentation based on the following specific use cases or scenarios:
- FFR computation (hemodynamic index computation): the FFR values are most sensitive with respect to the minimum radius values inside the coronary lesions. Other sensitive locations are the start and the end locations of the lesions. Hence, start and end stenosis markers and minimum radius locations would have a larger weight, while other locations, e.g., healthy segments, and stenosis regions with decreasing/increasing radius, would have a smaller weight.
- QCA: As for FFR only the start and end locations of the stenoses and the minimum radius locations would have a larger weight. In the case of FFR, all lesions are of interest, whereas in the case of QCA, typically the focus lies on the main stenosis. Hence, the weights for a secondary or tertiary lesion would be small.
- PCI planning: To correctly choose the stent size, it is important to assess the vessel diameter in the proximal and distal healthy segments (these locations would have large weights). In this case, the locations inside the stenosis, including the minimal radius location would have a small weight. Hence, as part of the user interaction workflow, the user would specify the main scope of the segmentation, and then the local uncertainty would be adapted. Additional input may be provided by a lesion detection module, to determine the start and end markers of the stenoses, the healthy segments, etc.

According to various examples, said adjusting of the respective segments of the contour may be performed using a trained machine learning algorithm.

The adjusting or editing operations described above could be designed as a machine learning based interactive editing or adjusting. A user can still provide input(s) by clicking on the point on the centerline and/or on the true lumen boundary. A machine learning algorithm can be trained which takes intensity images, initial segmentation results, and the user input, for example as a multi-channel input for a convolutional neural network, and outputs updated segmentation. The user input could be heatmaps or distance maps generated using the user click(s) and the computed uncertainty at that location (for example uncertainty could be used as the variance of the heatmap).

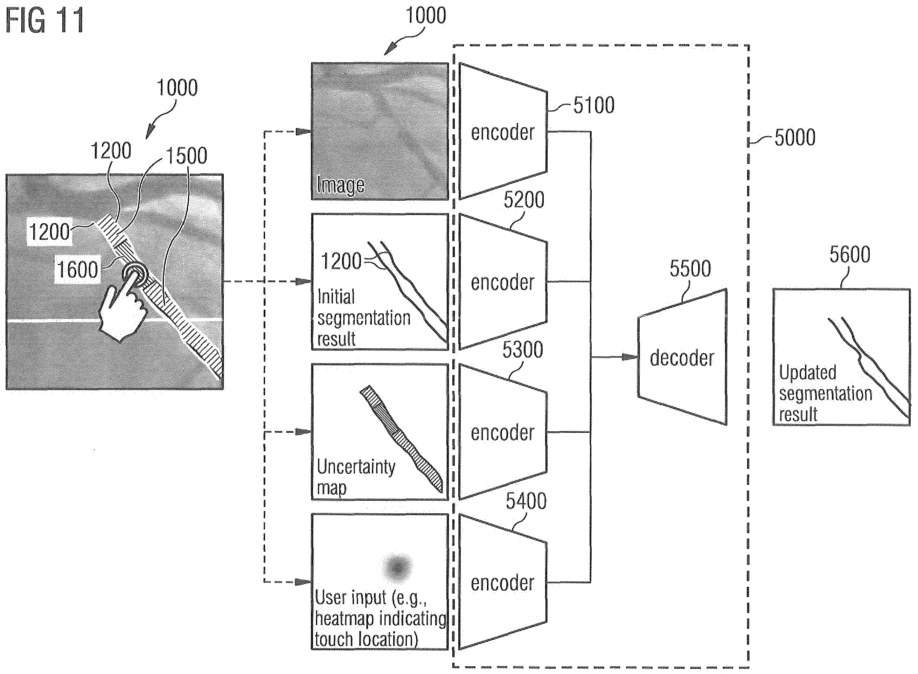

FIG. 11 schematically illustrates an exemplary machine learning algorithm 5000, e.g., a neural network, according to various examples. The machine learning algorithm may be configured to adjust the respective segment of the contour 1200 based on the respective magnitudes of one or more local segmentation uncertainties.

For example, during a PCI, a cardiologist may review the contour 1200 displayed on the screen of the display 860 of the C-arm machine 800 of FIG. 1. If the cardiologist is not satisfied with the contour 1200, the cardiologist may trigger execution of the machine learning algorithm 5000, e.g., by touching or clicking at a specific location where the contour 1200 may need to be adjusted. Then, a computing device of the C-arm machine 800 may automatically obtain inputs for the machine learning algorithm 500. Such inputs may comprise one or more frames of the multiple cardiac images obtained at blook 3100, an image of the contour 1200, an uncertainty map indicating respective magnitudes of local segmentation uncertainties associated with the portion C of the coronary arteries, and/or a heatmap indicating the specific location where the contour 1200 may need to be adjusted.

As shown in FIG. 11, the uncertainty map indicating respective magnitudes of local segmentation uncertainties associated with the portion C of the coronary arteries may comprise low uncertainty regions 1500 and high uncertainty regions. For example, the low uncertainty regions 1500 may comprise regions where the respective magnitudes of local segmentation uncertainties are smaller than a predefined threshold, while the high uncertainty regions may comprise regions where the respective magnitudes of local segmentation uncertainties are equal to or larger than the predefined threshold.

According to various examples, the machine learning algorithm 5000 may comprise multiple encoders 5100, 5200, 5300, and 5400, which respectively take one or more frames of the multiple cardiac images obtained at blook 3100, an image of the contour 1200, an uncertainty map indicating respective magnitudes of local segmentation uncertainties associated with the portion C of the coronary arteries, and a heatmap indicating the specific location where the contour 1200 may need to be adjusted as inputs. The respective outputs of the multiple encoders 5100, 5200, 5300, and 5400 may be combined or concatenated as input to a single decoder 5500, and the decoder 5500 outputs an image 5600 depicting an adjusted or updated contour of an updated lumen segmentation of the portion of the coronary arteries. The image 5600 may be displayed on the screen of the display 860 of the C-arm machine 800 of FIG. 1.

According to various examples, if the user such as a cardiologist is still not satisfied with the adjusted or updated contour depicting in the image 5600. The user can manually adjust or edit the adjusted contour. Such manual adjustment can be used to fine-tune parameters of the machine learning algorithm 5000 through a training process. In such a way, localized versions for individual clinicians or hospitals could be generated.

According to various examples, each of the multiple encoders 5100, 5200, 5300, and 5400 may be based on a convolutional neural network or a transformer.

In general, the machine learning algorithm 5000 can be trained using supervised learning, semi-supervised learning, or un-supervised learning. When using supervised learning, training examples may be represented as tuples (x, y). Here, x may include various inputs like original cardiac images, initial segmentation outcomes, uncertainty maps, and user inputs. The goal is to predict updated segmentation results such as the image 5600, which may be manually annotated as y. The training process may employ common optimizers like Adam or SGD along with predefined hyperparameters.

According to various examples, like those shown in FIG. 10B or FIG. 11, the method 3000 may further comprise visualizing, in a cardiac image comprising the given lumen segmentation of the portion of the coronary arteries, the respective magnitudes of the one or more local segmentation uncertainties.

According to this disclosure, the multiple cardiac images may comprise cardiac images acquired under different acquisition angles. If the correspondence between certain landmarks on the target coronary vessel, e.g., the portion C of coronary arteries, is given in the multiple cardiac images or views via user input or an adequate multi-view vessel matching algorithm, the techniques described herein may further take into account the radius profiles from multiple views, where each view contributes one or more frames to the co-registration/registration procedure. More comprehensive co-registration methodologies may be employed to compensate for e.g., different lengths of the target vessel (due to projection geometry).

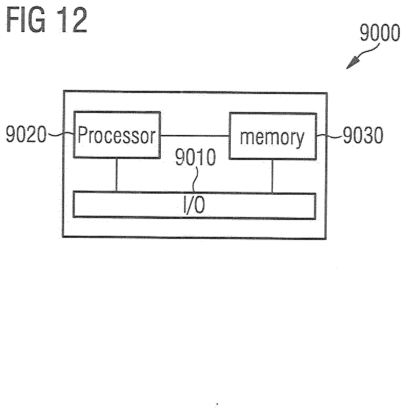

FIG. 12 is a block diagram of a computing device 9000 according to various examples. The computing device 9000 may comprise a processor 9020, a memory 9030, and an input/output interface 9010. The processor 9020 is configured to load program code from the memory 9030 and execute the program code. Upon executing the program code, the processor 9020 performs either the method 3000 and/or the method 4000 for processing multiple cardiac images depicting the same coronary artery of interest, e.g., the portion C of coronary arteries within an anatomical region of interest.

The computing device 9000 may be embedded in or connected with an angiography device such as the C-arm machine 800. I.e., the angiography device comprising the computing device 9000 may be configured to perform the method 3000 and/or the method 4000.

Referring to FIG. 1 again, the C-arm machine 800 may further comprise the computing device 9000 configured to perform the method 3000 and/or the method 4000. The computing device 9000 may be the C arm control unit 810 and/or the control panel 850. I.e., the computing device 9000 may be embedded in or connected with the C-arm machine 800, and thereby the C-arm machine 800 may be also configured to perform the method 3000 and/or the method 4000.

According to this disclosure, the method 4000 can be executed independently to determine respective magnitudes of one or more local segmentation uncertainties by processing multiple cardiac images. Additionally or optionally, the method 3000 can utilize the method 4000 as an exemplary realization of block 3200.

Summarizing, techniques have been described that facilitate efficient editing, adjusting, or manipulating of a proposed contour of a (given) lumen segmentation of a portion of coronary arteries by taking lumen information from multiple time points of a cardiac cycle into account. By processing multiple cardiac images, e.g., acquired in a cardiac cycle, magnitudes of one or more local segmentation uncertainties associated with respective segments of a contour of a given lumen segmentation of a portion of coronary arteries can be precisely determined, and thereby the respective segments of the contour can be finely and precisely adjusted or edited based on the respective magnitudes of the one or more local segmentation uncertainties. For example, the respective magnitudes of the one or more local segmentation uncertainties can be determined taking lumen radius variability during an entire cardiac cycle into account.

According to various examples, by leveraging information extracted from multiple cardiac images, the adjusting or editing process can be guided and thus accelerated. Additionally or optionally, by leveraging uncertainty stemming from an automatic processing and segmentation pipeline, a robust smart local editing model can be built.

According to the disclosure, a simple user interface can be designed for editing a contour of a lumen segmentation. Editing can be performed via a simple, touch-friendly user interface as indicated in FIG 10 or FIG. 11, which could potentially enable its use in environments, where precise mouse interaction for manual contour editing or similar approaches would be infeasible: possibly at the operating table on a touch-enabled device (tablet).

According to various examples, the techniques disclosed herein can be integrated into a multi-stage editing process: e.g., first use proposed uncertainty-guided editing for fast local and global adjustment of contours, then "normal" manual editing for fine details, that were not captured by the proposed uncertainty-guided editing.

The techniques disclosed herein may further increase transparency. Instead of only using the localized uncertainty as a means to guide the editing, it could be displayed to the user, e.g., via color-coding of the contour or centerline, and thus visually indicate regions along the target vessel, where the automatic contours are more likely to require user editing because aggregated contours may have low confidence.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

For illustration, the disclosure is explained in detail based on uncertainty derived from variabilities across segmentation from multiple frames. However, other sources of uncertainty can be further taken into account. For example, the uncertainty could be derived from localized image quality measures or even input by the user.

According to various examples, different use cases or utilizations of the contour of the lumen segmentation, e.g., computation of FFR, QCA, or PCI planning, may be not impacted equally by different local segments or sections of the coronary artery tree. Focusing human review and editing on regions that will most affect the subsequent processing of the contour can significantly accelerate the review process.

For illustration, the disclosure is explained in detail based on X-ray angiography, the techniques disclosed herein can be also applied to angiography that may be done using scans instead of X-rays, such as CT (computed tomography) angiography or MRI (Magnetic resonance imaging) angiography.

Further, the approaches described herein may be used also when certain parts of the region of interest are not well visible on a certain frame, e.g., due to foreshortening, vessel overlap, etc.

In addition, the approaches described herein may be used also when the multiple cardiac images are obtained from different angiography views / acquired under different acquisition angles. Certain regions of interest may be recommended depending on the angulation of the acquisition (e.g. LM/pLAD/proximal LCx for the spider view). By combining the recommended regions of interest from the various angulations, a more accurate representation of the entire region of interest may be obtained.

Further, the approaches described herein may be used also when the multiple cardiac images are obtained from different imaging modalities: OCT / IVUS: highly accurate anatomical information on a single vessel/vessel segment or CCTA: entire coronary arterial tree modeled in 3D.

For example, if intravascular imaging (IVUS, OCT) is available for certain coronary segments and the data can be co-registered, the uncertainty may be reduced inside those segments. High-resolution CTA data may also be used to reduce uncertainty within vessel segments with e.g., overlap, low image quality, etc.

## Claims

1. A computer-implemented method (3000), comprising:
- obtaining (3100) multiple cardiac images (f1-f10), each of the multiple cardiac images (f1-f10) depicting a portion (C) of coronary arteries within an anatomical region of interest;
- determining (3200), based on the multiple cardiac images (f1-f10), respective magnitudes of one or more local segmentation uncertainties, wherein each of the one or more local segmentation uncertainties is associated with a respective segment of a contour (1200) of a given lumen segmentation of the portion (C) of the coronary arteries;
- adjusting (3300) the respective segments of the contour (1200) based on the respective magnitudes of the one or more local segmentation uncertainties;
**characterised in that**
said determining (3200) of the respective magnitudes of the one or more local segmentation uncertainties comprises:
- determining (4200), based on each of the multiple cardiac images (f1-f10), a respective set of lumen radius measurements comprising multiple lumen radius measurements respectively associated with multiple locations (1101-1124) of the portion (C) of the coronary arteries;
- determining (4300), based on the respective sets of lumen radius measurements, a maximum stenosis severity profile (2100) and a minimum stenosis severity profile (2200) associated with the portion (C) of the coronary arteries;
- determining (4400) the respective magnitudes of the one or more local segmentation uncertainties based on the maximum stenosis severity profile (2100) and the minimum stenosis severity profile (2200).

2. The computer-implemented method (3000) of claim 1, said determining (4300) of the maximum stenosis severity profile (2100) and the minimum stenosis severity profile (2200) comprising:
- determining a reference set of the lumen radius measurements;
- aligning each of the respective sets (p1-p10) of the lumen radius measurements with the reference set of the lumen radius measurements;
- determining the maximum stenosis severity profile (2100) and the minimum stenosis severity profile (2200) based on the respective aligned sets (p1'-p10') of the lumen radius measurements.

3. The computer-implemented method (3000) of claim 2, said determining of the reference set of the lumen radius measurements comprising:
- selecting the set of the lumen radius measurements having the longest length as the reference set of the lumen radius measurements.

4. The computer-implemented method (3000) of claim 3, said aligning of each of the respective sets (p1-p10) of the lumen radius measurements with the reference set of the lumen radius measurements comprising:
for each of the respective unaligned sets of the lumen radius measurements, iteratively performing the following steps:
- selecting the longest set of the lumen radius measurements among all the unaligned sets of the lumen radius measurements;
- aligning the selected set of the lumen radius measurements with the reference set of the lumen radius measurements;
- updating the reference set of the lumen radius measurements based on all aligned sets of the lumen radius measurements.

5. The computer-implemented method (3000) of any one of claims 2-4,
wherein said aligning is based on one or more anatomical landmarks within the anatomical region of interest.

6. The computer-implemented method (3000) of any one of claims 1-5,
the maximum stenosis severity profile (2100) and the minimum stenosis severity profile (2200) are respectively determined based on local concavities of multiple curves, each of the multiple curves depicting a respective relationship between a respective set of the lumen radius measurements and the multiple locations (1101-1124) of the portion (C) of the coronary arteries.

7. The computer-implemented method (3000) of any one of claims 1-6, said determining (4200) of the respective set of lumen radius measurements comprising:
- segmenting the portion (C) of the coronary arteries from the respective cardiac image of the multiple cardiac images (f1-f10); and,
- determining the respective set of the lumen radius measurements based on the respective segmented portion of the coronary arteries.

8. The computer-implemented method (3000) of any one of the preceding claims, wherein said adjusting (3300) of the respective segments of the contour (1200) comprises:
for each one of the one or more local segmentation uncertainties:
- determining whether the respective magnitude is greater than a predefined threshold;
- upon determining the respective magnitude is greater than the predefined threshold: adjusting the respective segment of the contour (1200).

9. The computer-implemented method (3000) of any one of the preceding claims,
wherein said adjusting (3300) of the respective segments of the contour (1200) is further based on a specific use case of the given lumen segmentation and /or an anatomical structure of the portion (C) of the coronary arteries.

10. The computer-implemented method (3000) of any one of the preceding claims,
wherein said adjusting (3300) of the respective segments of the contour is performed using a trained machine learning algorithm (5000).

11. The computer-implemented method (3000) of any one of the preceding claims, further comprising:
- visualizing, in a cardiac image comprising the given lumen segmentation of the portion of the coronary arteries, the respective magnitudes of the one or more local segmentation uncertainties.

12. The computer-implemented method (3000) of any one of the preceding claims, further comprising:
- determining the anatomical region of interest based on multiple predefined seed points.

13. The computer-implemented method (3000) of any one of the preceding claims, further comprising:
- registering the multiple cardiac images (f1-f10).

14. The computer-implemented method (3000) of any one of the preceding claims, said obtaining (3100) of the multiple cardiac images comprising:
- obtaining an angiogram acquired during an angiography exam of the anatomical region of interest;
- selecting, based on at least one pre-defined criterion, the multiple cardiac images (f1-f10) among frames of the angiogram.

15. The computer-implemented method (3000) of any one of the preceding claims,
wherein the multiple cardiac images (f1-f10) comprise cardiac images acquired under different acquisition angles.

16. The computer-implemented method (3000) of any one of the preceding claims,
wherein the multiple cardiac images (f1-f10) comprise cardiac images acquired using multi-phase coronary computed tomography angiography.

17. A computing device (9000), the device comprising a processor (9020) and a memory (9030), wherein upon loading and executing program code from the memory (9030), the processor (9020) is configured to perform the method (3000) of any one of the preceding claims.

18. An angiography device (800) comprising the computing device (9000) of claim 17.

## Patentansprüche

1. Computerimplementiertes Verfahren (3000), umfassend:
- Erhalten (3100) mehrerer Herzbilder (f1-f10), wobei jedes der mehreren Herzbilder (f1-f10) einen Abschnitt (C) von Koronararterien innerhalb einer anatomischen Interessensregion darstellt;
- Bestimmen (3200) jeweiliger Größen einer oder mehrerer lokaler Segmentierungsunsicherheiten basierend auf den mehreren Herzbildern (f1-f10), wobei jede der einen oder der mehreren lokalen Segmentierungsunsicherheiten mit einem jeweiligen Segment einer Kontur (1200) einer gegebenen Lumensegmentierung des Abschnitts (C) der Koronararterien assoziiert ist;
- Anpassen (3300) der jeweiligen Segmente der Kontur (1200) basierend auf den jeweiligen Größen der einen oder der mehreren lokalen Segmentierungsunsicherheiten;
**dadurch gekennzeichnet, dass**
das Bestimmen (3200) der jeweiligen Größen der einen oder der mehreren lokalen Segmentierungsunsicherheiten Folgendes umfasst:
- Bestimmen (4200) eines jeweiligen Satzes von Lumenradiusmessungen, der mehrere Lumenradiusmessungen umfasst, die jeweils mit mehreren Positionen (1101-1124) des Abschnitts (C) der Koronararterien assoziiert sind, basierend auf jedem der mehreren Herzbilder (f1-f10);
- Bestimmen (4300) eines Profils maximaler Stenoseschwere (2100) und eines Profils minimaler Stenoseschwere (2200), die mit dem Abschnitt (C) der Koronararterien assoziiert sind, basierend auf den jeweiligen Sätzen von Lumenradiusmessungen;
- Bestimmen (4400) der jeweiligen Größen der einen oder der mehreren lokalen Segmentierungsunsicherheiten basierend auf dem Profil maximaler Stenoseschwere (2100) und dem Profil minimaler Stenoseschwere (2200).

2. Computerimplementiertes Verfahren (3000) nach Anspruch 1, wobei das Bestimmen (4300) des Profils maximaler Stenoseschwere (2100) und des Profils minimaler Stenoseschwere (2200) Folgendes umfasst:
- Bestimmen eines Referenzsatzes der Lumenradiusmessungen;
- Abgleichen jedes der jeweiligen Sätze (p1-p10) der Lumenradiusmessungen mit dem Referenzsatz der Lumenradiusmessungen;
- Bestimmen des Profils maximaler Stenoseschwere (2100) und des Profils minimaler Stenoseschwere (2200) basierend auf den jeweiligen abgeglichenen Sätzen (p1'-p10') der Lumenradiusmessungen.

3. Computerimplementiertes Verfahren (3000) nach Anspruch 2, wobei das Bestimmen des Referenzsatzes der Lumenradiusmessungen Folgendes umfasst:
- Auswählen des Satzes der Lumenradiusmessungen mit der längsten Länge als Referenzsatz der Lumenradiusmessungen.

4. Computerimplementiertes Verfahren (3000) nach Anspruch 3, wobei das Abgleichen jedes der jeweiligen Sätze (p1-p10) der Lumenradiusmessungen mit dem Referenzsatz der Lumenradiusmessungen Folgendes umfasst:
für jeden der jeweiligen nicht abgeglichenen Sätze der Lumenradiusmessungen iteratives Ausführen der folgenden Schritte:
- Auswählen des längsten Satzes der Lumenradiusmessungen unter allen nicht abgeglichenen Sätzen der Lumenradiusmessungen;
- Abgleichen des ausgewählten Satzes der Lumenradiusmessungen mit dem Referenzsatz der Lumenradiusmessungen;
- Aktualisieren des Referenzsatzes der Lumenradiusmessungen basierend auf allen abgeglichenen Sätzen der Lumenradiusmessungen.

5. Computerimplementiertes Verfahren (3000) nach einem der Ansprüche 2-4,
wobei das Abgleichen auf einer oder mehreren anatomischen Landmarken innerhalb der anatomischen Interessensregion basiert.

6. Computerimplementiertes Verfahren (3000) nach einem der Ansprüche 1-5,
wobei das Profil maximaler Stenoseschwere (2100) und das Profil minimaler Stenoseschwere (2200) jeweils basierend auf lokalen Konkavitäten mehrerer Kurven bestimmt werden, wobei jede der mehreren Kurven eine jeweilige Beziehung zwischen einem jeweiligen Satz der Lumenradiusmessungen und den mehreren Positionen (1101-1124) des Abschnitts (C) der Koronararterien darstellt.

7. Computerimplementiertes Verfahren (3000) nach einem der Ansprüche 1-6, wobei das Bestimmen (4200) des jeweiligen Satzes von Lumenradiusmessungen Folgendes umfasst:
- Segmentieren des Abschnitts (C) der Koronararterien aus dem jeweiligen Herzbild der mehreren Herzbilder (f1-f10); und
- Bestimmen des jeweiligen Satzes der Lumenradiusmessungen basierend auf dem jeweiligen segmentierten Abschnitt der Koronararterien.

8. Computerimplementiertes Verfahren (3000) nach einem der vorhergehenden Ansprüche, wobei das Anpassen (3300) der jeweiligen Segmente der Kontur (1200) Folgendes umfasst:
für jede der einen oder mehreren lokalen Segmentierungsunsicherheiten:
- Bestimmen, ob die jeweilige Größe größer als ein vordefinierter Schwellenwert ist;
- wenn bestimmt wird, dass die jeweilige Größe größer als der vorgegebene Schwellenwert ist: Anpassen des jeweiligen Segments der Kontur (1200).

9. Computerimplementiertes Verfahren (3000) nach einem der vorhergehenden Ansprüche,
wobei das Anpassen (3300) der jeweiligen Segmente der Kontur (1200) ferner auf einem spezifischen Anwendungsfall der gegebenen Lumensegmentierung und/oder einer anatomischen Struktur des Abschnitts (C) der Koronararterien basiert.

10. Computerimplementiertes Verfahren (3000) nach einem der vorhergehenden Ansprüche,
wobei das Anpassen (3300) der jeweiligen Segmente der Kontur unter Verwendung eines trainierten Algorithmus für maschinelles Lernen (5000) durchgeführt wird.

11. Computerimplementiertes Verfahren (3000) nach einem der vorhergehenden Ansprüche, ferner umfassend:
- Visualisieren der jeweiligen Größen der einen oder der mehreren lokalen Segmentierungsunsicherheiten in einem Herzbild, das die gegebene Lumensegmentierung des Abschnitts der Koronararterien umfasst.

12. Computerimplementiertes Verfahren (3000) nach einem der vorhergehenden Ansprüche, ferner umfassend:
- Bestimmen der anatomischen Interessensregion basierend auf mehreren vordefinierten Startpunkten.

13. Computerimplementiertes Verfahren (3000) nach einem der vorhergehenden Ansprüche, ferner umfassend:
- Registrieren der mehreren Herzbilder (f1-f10).

14. Computerimplementiertes Verfahren (3000) nach einem der vorhergehenden Ansprüche, wobei das Erhalten (3100) der mehreren Herzbilder Folgendes umfasst:
- Erhalten eines Angiogramms, das während einer Angiographieuntersuchung der interessierenden anatomischen Region aufgenommen wurde;
- Auswählen der mehreren Herzbilder (f1-f10) unter Einzelbildern des Angiogramms basierend auf mindestens einem vordefinierten Kriterium.

15. Computerimplementiertes Verfahren (3000) nach einem der vorhergehenden Ansprüche,
wobei die mehreren Herzbilder (f1-f10) Herzbilder umfassen, die unter verschiedenen Aufnahmewinkeln aufgenommen wurden.

16. Computerimplementiertes Verfahren (3000) nach einem der vorhergehenden Ansprüche,
wobei die mehreren Herzbilder (f1-f10) Herzbilder umfassen, die unter Verwendung einer mehrphasige Computertomographie-Koronarangiographie aufgenommen wurden.

17. Computervorrichtung (9000), wobei die Vorrichtung einen Prozessor (9020) und einen Speicher (9030) umfasst, wobei der Prozessor (9020) beim Laden und Ausführen von Programmcode aus dem Speicher (9030) dazu ausgelegt ist, das Verfahren (3000) nach einem der vorhergehenden Ansprüche durchzuführen.

18. Angiographievorrichtung (800), umfassend eine Computervorrichtung (9000) nach Anspruch 17.

## Revendications

1. Procédé (3000) mis en œuvre par ordinateur, le procédé comprenant les étapes suivantes :
- obtenir (3100) plusieurs images cardiaques (f1-f10), chacune des multiples images cardiaques (f1-f10) représentant une partie (C) d'artères coronaires au sein d'une région anatomique d'intérêt ;
- déterminer (3200), à partir des multiples images cardiaques (f1-f10), des grandeurs respectives d'une ou plusieurs incertitudes de segmentation locales, où chacune des une ou plusieurs incertitudes de segmentation locales est associée à un segment respectif d'un contour (1200) d'une segmentation de lumière donnée de la partie (C) des artères coronaires ;
- ajuster (3300) les segments respectifs du contour (1200) sur la base des grandeurs respectives des une ou plusieurs incertitudes de segmentation locales ;
**caractérisé en ce que**
ladite détermination (3200) des grandeurs respectives des une ou plusieurs incertitudes de segmentation locales comprend les étapes suivantes :
- déterminer (4200), sur la base de chacune des multiples images cardiaques (f1-f10), un ensemble respectif de mesures de rayon de lumière comprenant plusieurs mesures de rayon de lumière associées respectivement à plusieurs localisations (1101-1124) de la partie (C) des artères coronaires,
- déterminer (4300), sur la base des ensembles respectifs de mesures de rayon de lumière, un profil de sévérité de sténose maximale (2100) et un profil de sévérité de sténose minimale (2200) associé à la partie (C) des artères coronaires ;
- déterminer (4400) les grandeurs respectives des une ou plusieurs incertitudes de segmentation locales sur la base du profil de sévérité de sténose maximale (2100) et du profil de sévérité de sténose minimale (2200).

2. Procédé (3000) mis en œuvre par ordinateur selon la revendication 1, ladite détermination (4300) du profil de sévérité de sténose maximale (2100) et du profil de sévérité de sténose minimale (2200) comprenant les étapes suivantes :
- déterminer un ensemble de référence des mesures de rayon de lumière,
- aligner chacun des ensembles respectifs (p1-p10) des mesures de rayon de lumière avec l'ensemble de référence des mesures de rayon de lumière ;
- déterminer le profil de sévérité de sténose maximale (2100) et le profil de sévérité de sténose minimale (2200) sur la base des ensembles alignés respectifs (p1'-p10') des mesures de rayon de lumière.

3. Procédé (3000) mis en œuvre par ordinateur selon la revendication 2, ladite détermination de l'ensemble de référence des mesures de rayon de lumière comprenant l'étape suivante :
- sélectionner l'ensemble des mesures de rayon de lumière ayant la plus grande longueur comme ensemble de référence des mesures de rayon de lumière.

4. Procédé (3000) mis en œuvre par ordinateur selon la revendication 3, ledit alignement de chacun des ensembles respectifs (p1-p10) des mesures de rayon de lumière avec l'ensemble de référence des mesures de rayon de lumière comprenant :
pour chacun des ensembles respectifs non alignés de mesures de rayon de lumière, d'exécuter de manière itérative les étapes suivantes :
- sélectionner l'ensemble le plus long de mesures de rayon de lumière parmi tous les ensembles non alignés des mesures de rayon de lumière ;
- aligner l'ensemble sélectionné de mesures de rayon de lumière sur l'ensemble de référence des mesures de rayon de lumière ;
- mettre à jour l'ensemble de référence des mesures de rayon de lumière sur la base de tous les ensembles alignés des mesures de rayon de lumière.

5. Procédé mis en œuvre par ordinateur (3000) selon l'une quelconque des revendications 2 à 4,
dans lequel ledit alignement est basé sur un ou plusieurs repères anatomiques à l'intérieur de la région anatomique d'intérêt.

6. Procédé mis en œuvre par ordinateur (3000) selon l'une quelconque des revendications 1 à 5,
le profil de sévérité de sténose maximale (2100) et le profil de sévérité de sténose minimale (2200) étant respectivement déterminés sur la base de concavités locales de courbes multiples, chacune des courbes multiples représentant une relation respective entre un ensemble respectif de mesures de rayon de lumière et les multiples emplacements (1101-1124) de la partie (C) des artères coronaires.

7. Procédé mis en œuvre par ordinateur (3000) selon l'une quelconque des revendications 1 à 6, ladite détermination (4200) de l'ensemble respectif de mesures de rayon de lumière comprenant les étapes suivantes :
- segmenter la partie (C) des artères coronaires à partir de l'image cardiaque respective des multiples images cardiaques (f1-f10) ; et
- déterminer l'ensemble respectif des mesures de rayon de lumière sur la base de la partie segmentée respective des artères coronaires.

8. Procédé mis en œuvre par ordinateur (3000) selon l'une quelconque des revendications précédentes, dans lequel ledit ajustement (3300) des segments respectifs du contour (1200) comprend les étapes suivantes :
pour chacune des une ou plusieurs incertitudes de segmentation locales :
- déterminer si l'amplitude respective est supérieure à un seuil prédéfini ;
- lors de la détermination que l'amplitude respective est supérieure au seuil prédéfini, ajuster le segment respectif du contour (1200).

9. Procédé mis en œuvre par ordinateur (3000) selon l'une quelconque des revendications précédentes,
dans lequel ledit ajustement (3300) des segments respectifs du contour (1200) est en outre fondé sur un cas d'utilisation spécifique de la segmentation de lumière donnée et/ou une structure anatomique de la partie (C) des artères coronaires.

10. Procédé mis en œuvre par ordinateur (3000) selon l'une quelconque des revendications précédentes,
dans lequel ledit ajustement (3300) des segments respectifs du contour est réalisé à l'aide d'un algorithme d'apprentissage machine entraîné (5000).

11. Procédé mis en œuvre par ordinateur (3000) selon l'une quelconque des revendications précédentes, comprenant en outre l'étape suivante :
- visualiser, sur une image cardiaque comprenant la segmentation de lumière donnée de la partie des artères coronaires, les grandeurs respectives des une ou plusieurs incertitudes de segmentation locales.

12. Procédé mis en œuvre par ordinateur (3000) selon l'une quelconque des revendications précédentes, comprenant en outre l'étape suivante :
- déterminer la région anatomique d'intérêt en fonction de multiples points de départ prédéfinis.

13. Procédé mis en œuvre par ordinateur (3000) selon l'une quelconque des revendications précédentes, comprenant en outre l'étape suivante :
- recaler les multiples images cardiaques (f1-f10).

14. Procédé mis en œuvre par ordinateur (3000) selon l'une quelconque des revendications précédentes, ladite obtention (3100) des multiples images cardiaques comprenant les étapes suivantes :
- obtenir un angiogramme acquis au cours d'un examen angiographique de la région anatomique d'intérêt ;
- sélectionner, en fonction d'au moins un critère prédéfini, les multiples images cardiaques (f1-f10) parmi des trames de l'angiogramme.

15. Procédé mis en œuvre par ordinateur (3000) selon l'une quelconque des revendications précédentes,
dans lequel les images cardiaques (f1-f10) comprennent des images cardiaques acquises sous des angles d'acquisition différents.

16. Procédé mis en œuvre par ordinateur (3000) selon l'une quelconque des revendications précédentes,
dans lequel les multiples images cardiaques (f1-f10) comprennent des images cardiaques acquises à l'aide d'une angiographie coronarienne par tomodensitométrie multiphase.

17. Dispositif informatique (9000), le dispositif comprenant un processeur (9020) et une mémoire (9030), dans lequel, lors du chargement et de l'exécution d'un code de programme à partir de la mémoire (9030), le processeur (9020) est configuré pour exécuter le procédé (3000) selon l'une quelconque des revendications précédentes.

18. Dispositif d'angiographie (800) comprenant le dispositif informatique (9000) selon la revendication 17.
